# FASCICULE DE BREVET EUROPEEN

(11) **EP 1 037 576 B1**
(45) Date de publication et mention de la délivrance du brevet: **10.03.2004**
(21) Numéro de dépôt: 98959968.3
(22) Date de dépôt: 11.12.1998
(51) Int. Cl.: A61F 2/38

(54) **PROTHESE DE GENOU**
KNIEPROTHESE
KNEE PROSTHESIS

(30) Priorité: 12.12.1997 FR 9716044
(43) Date de publication de la demande: 27.09.2000
(60) Demande divisionnaire: 03011733.7
(73) Titulaire: Tornier SA, 38330 Saint Ismier (FR)
(72) Inventeur: TORNIER, Alain, F-38330 Saint Ismier (FR)
(74) Mandataire: Schmitt, John
(86) Numéro de dépôt international: PCT/FR1998/002706
(87) Numéro de publication internationale: WO 1999/030650

(56) Documents cités:
- EP-A- 0 346 183
- US-A- 5 358 527
- US-A- 5 658 342

## Description

La présente invention est relative à des perfectionnements apportés aux prothèses totales de genou, comportant à la manière connue, un élément fémoral et un plateau tibial. L'élément fémoral est utilisé pour remplacer les condyles détruits du fémur, tandis que le plateau tibial se substitue à la partie supérieure endommagée du tibia.

Il existe déjà des prothèses de ce genre qui sont tout d'abord des prothèses à glissement, avec ou sans conservation du ligament postérieur croisé, dans laquelle l'élément fémoral glisse sur une zone de faible surface par rapport au plateau tibial.

Les caractéristiques du préambule de la revendication 1 sont connues du document US-A-5 358 527.

On connaît par le document GB-A-2 067 412, une prothèse de genou dont l'élément fémoral et pourvu entre ses deux condyles d'un passage se terminant par une came convexe. Le plateau tibial, de la prothèse considérée, comporte des empreintes de réception des condyles et d'une rampe concave s'élevant vers l'élément fémoral et pénétrant largement entre ses deux condyles.

Lors de la coopération de la came avec la rampe et après un déplacement angulaire libre, il se crée des contraintes considérables, du fait du bras de levier important, créé par rapport à la fixation du plateau tibial sur la partie supérieure du tibia.

De plus, l'importance de la pénétration de la rampe du plateau tibial limite considérablement l'amplitude de la rotation du tibia par rapport au fémur.

Enfin la hauteur de la rampe du plateau tibial entraîne une résection importante de l'épiphyse du fémur, et qui bien entendu diminue considérablement sa résistance.

Ces inconvénients ont été résolus dans le brevet européen N° 0 294 298 appartenant au demandeur. La prothèse totale de genou décrite dans ce document, comprend un élément fémoral muni de deux condyles asymétriques et d'une came convexe qui est disposée entre les deux condyles et réalisée sous la forme d'un troisième condyle.

Le plateau tibial comporte une piste horizontale recevant la came convexe de l'élément fémoral qui est situé en dessous du niveau des empreintes sur lesquelles glissent les condyles de l'élément fémoral. Le plateau tibial comprend, entre les empreintes et dans l'alignement de la piste, une rampe concave affectant la forme d'une butée courbe qui permet que le point de contact de la came se trouve à une faible distance du plateau tibial sur toute la course de contact de ces deux éléments, de manière à diminuer au maximum les contraintes sur ledit plateau tibial.

Du fait de la position basse de la came ou troisième condyle, on diminue au maximum le bras de levier créé par son action contre la rampe ou butée du plateau tibial, de sorte que les contraintes transmises au tibia sont minimes.

Les prothèses totales de genou décrites ci-dessus comportent certains inconvénients en ce qui concerne l'impossibilité de conserver le ligament croisé postérieur du fait de l'emplacement central de la came convexe entre les deux condyles.

En effet, le fémur d'un genou sain est composé de deux condyles distaux / postérieurs qui sont séparés par un espace permettant le passage du ligament croisé postérieur stabilisant l'articulation en position postérieure.

Les perfectionnements apportés à la prothèse de genou suivant la présente invention ont pour objet de conserver le passage du ligament croisé postérieur en gardant les avantages de la came convexe décrite dans le brevet européen N° 0 294 298.

La prothèse totale de genou suivant la présente invention comprend un élément fémoral pourvu de deux condyles, un externe, un interne, dont l'un au moins est solidaire sur l'un de ses côtés d'une came convexe assurant la stabilisation postérieure et un plateau tibial comportant deux empreintes propres à recevoir lesdits condyles et au moins une piste disposée à proximité immédiate de l'empreinte recevant le condyle solidaire de la came convexe, ladite piste étant délimitée par au moins une butée courbe et au moins une empreinte horizontale 19, 24 entre les extrémités postérieures et antérieures du plateau tibial, tandis qu'entre les deux condyles de l'élément fémoral et les deux empreintes de plateau tibial sont prévus respectivement un espace libre et une échancrure pour le passage du ligament croisé postérieur du genou.

La prothèse totale de genou conforme à la présente invention comprend sur le côté interne d'un condyle de l'élément fémoral, une came convexe réalisée sous la forme d'un troisième condyle de manière à délimiter les dimensions de l'espace libre communiquant avec l'échancrure du plateau tibial pour le passage du ligament croisé postérieur du genou, tandis que la came convexe coopère avec une piste située entre et en dessous du niveau des empreintes recevant lesdits condyles.

La prothèse totale de genou comprend sur le côté externe d'un condyle de l'élément fémoral, une came convexe réalisée sous la forme d'un troisième condyle de manière que l'espace libre soit délimité entre le condyle externe et le condyle interne pour communiquer avec l'échancrure du plateau tibial pour le passage du ligament croisé postérieur du genou, tandis que la came convexe coopère avec une piste située à l'extérieur et en dessous du niveau de l'empreinte recevant le condyle.

La prothèse totale de genou suivant la présente invention comporte sur les côtés internes de chaque condyle de l'élément fémoral une came convexe réalisée sous la forme d'un troisième condyle de manière à délimiter entre lesdites cames disposées à l'opposé l'une de l'autre, les dimensions de l'espace libre communiquant avec l'échancrure du plateau tibial pour le passage du ligament croisé postérieur du genou, tandis que chaque came convexe coopère avec une piste située entre et en dessous du niveau des empreintes recevant lesdits condyles.

La prothèse totale de genou suivant la présente invention comprend un plateau tibial pourvu d'au moins une piste recevant la came convexe correspondante de l'élément fémoral et qui comporte du côté de l'extrémité postérieure du plateau une empreinte horizontale située en dessous du niveau des empreintes propres à recevoir le condyle, ladite empreinte se prolongeant vers l'extrémité antérieure dudit plateau par une butée courbe qui se dresse à l'opposé du tibia.

La prothèse totale de genou suivant la présente invention comprend un plateau tibial pourvu d'au moins une piste recevant la came convexe correspondante de l'élément fémoral et qui comporte du côté de l'extrémité postérieure du plateau une première butée courbe s'élevant légèrement au-dessus des empreintes prévues pour les condyles, ladite première butée se prolongeant vers l'extrémité antérieure dudit plateau par une seconde butée courbe qui se dresse à l'opposé du tibia.

La prothèse totale de genou suivant la présente invention comprend un plateau tibial pourvu d'au moins une piste recevant la came convexe correspondante de l'élément fémoral et qui comporte du côté de l'extrémité postérieure du plateau une butée courbe s'élevant légèrement au-dessus des empreintes prévues pour les condyles, ladite butée se prolongeant vers l'extrémité antérieure dudit plateau par une empreinte horizontale située en dessous du niveau desdites empreintes.

La prothèse totale de genou suivant la présente invention comprend une came qui présente un profil courbe s'étendant à partir du voile jusqu'à l'aile antérieure de l'élément fémoral.

La prothèse totale de genou suivant la présente invention comprend une came qui présente un profil en portion de tore.

La prothèse totale de genou suivant la présente invention comprend une came qui présente une largeur qui est inférieure à celle prévue pour les condyles externe et interne.

La description qui va suivre en regard des dessins annexés, donnés à titre d'exemples non limitatifs, permettra de mieux comprendre l'invention, les caractéristiques qu'elle présente et les avantages qu'elle est susceptible de procurer:
Figure 1 est une vue illustrant la prothèse totale de genou suivant la présente invention.
Figures 2 et 3 sont des vues montrant des variantes de la prothèse de genou suivant la présente invention.
Figure 4 est une vue représentant le glissement de l'élément fémoral sur le plateau tibial de la prothèse totale de genou suivant la présente invention.
Figures 5 et 6 sont des vues illustrant des variantes de plateau tibial pour le glissement de l'élément fémoral de la prothèse totale de genou. La figure 5 ne montre pas une variante de la prothèse totale de genou suivant la présente invention.

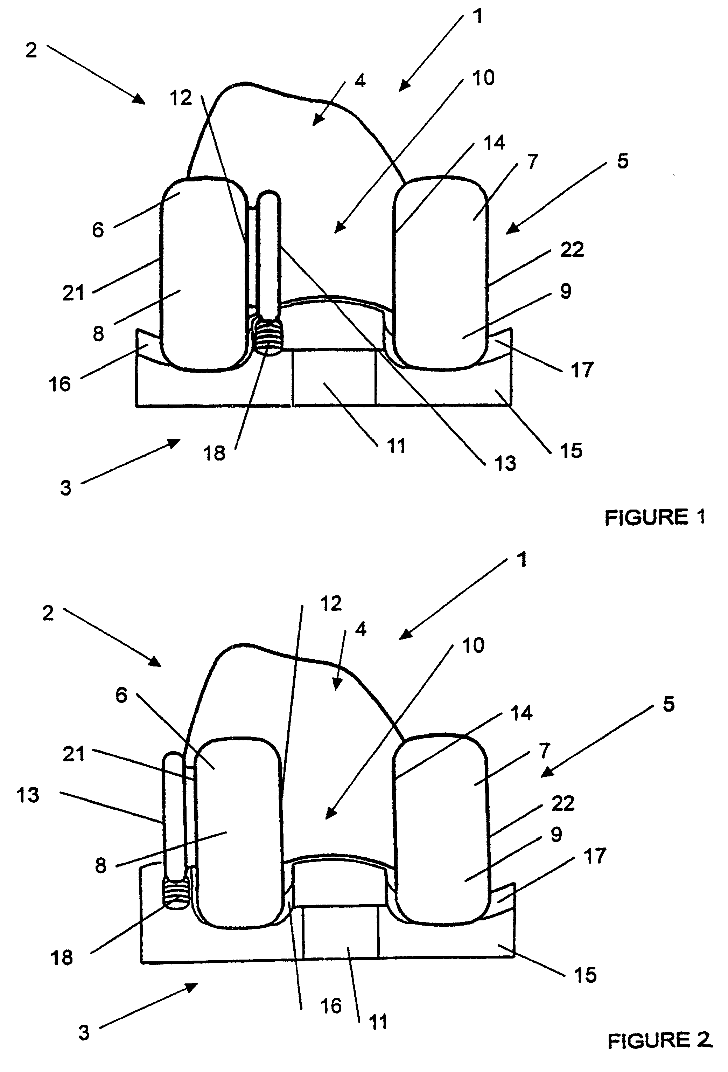

En figure 1 on a montré une prothèse totale du genou 1 qui comprend essentiellement un élément fémoral 2 et un plateau tibial 3.

L'élément fémoral 2 comporte en section transversale la forme générale d'un U, c'est à dire qu'il est formé d'une première aile antérieure 4 se prolongeant par une seconde aile postérieure 5.

Les faces internes planes de ses deux ailes sont destinées à coopérer avec des méplats ménagés au moyen de découpes antérieure et postérieure réalisées sur l'épiphyse du fémur non représenté.

L'aile postérieure 5 est constituée de deux voiles distincts et parallèles 6 et 7 solidaires du voile antérieure 4. La face externe des voiles 6 et 7 et de l'aile antérieure 4 présente un profil courbe définissant deux condyles latéraux 8 et 9 qui peuvent être symétrique ou asymétrique suivant l'utilisation de la prothèse 1.

Le condyle externe 8 est séparé du condyle interne 9 par un espace libre 10 qui communique avec un espace ou une échancrure 11 ménagée dans la partie postérieure du plateau tibial 3 pour le passage du ligament croisé postérieur non représenté.

Le condyle externe 8 comporte sur son côté interne 12 et dans l'espace libre 10 une came convexe 13 formant un troisième condyle. La came 13 présente un profit courbe s'étendant à partir du voile 6 jusqu'à l'aile antérieure 4.

La came convexe 13 solidaire du condyle externe 8 délimite avec le côté opposé et interne 14 du condyle interne 9 les dimensions de l'espace libre 10 prévu pour le passage du ligament croisé.

Le plateau tibial 3 est constitué d'un élément en matière plastique 15 à très faible coefficient de frottement destiné à être engagé sur une plaque métallique solidaire d'une queue, non représenté, qui vient s'ancrer dans le tibia.

L'élément 15 est creusé de part et d'autre de l'échancrure 11 de deux empreintes 16 et 17 recevant respectivement les condyles 8 et 9 de l'élément fémoral 2 pour permettre une rotation glissement du genou artificiel 1.

Entre les deux empreintes 16 et 17 et à proximité de celle 16, est ménagé une piste 18 destinée à recevoir la came convexe 13 solidaire du condyle externe 8.

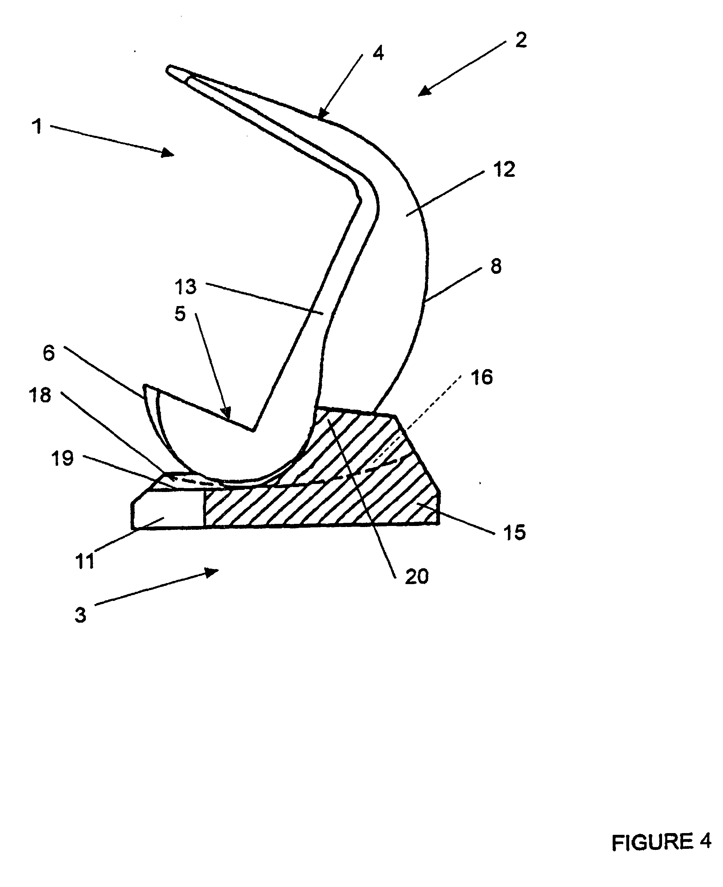

Le profil de la piste 18 peut varier en fonction de l'utilisation de la prothèse totale du genou 1, comme cela est montré en figures 4 à 6.

De la même manière que pour le condyle externe 8, le condyle interne 9 peut être solidaire sur son côté interne 14 d'une came 13 coopérant avec une piste 18 ménagée entre les deux empreintes 16 et 17, et à proximité de celle 17.

La came convexe 13, solidaire du côté interne 12 du condyle 8, permet de favoriser le mouvement anatomique de la prothèse 1, puisque le condyle externe 8 recule plus que l'autre par rapport au plateau tibial 3.

En figure 2 on a représenté le condyle externe 8 de l'élément fémoral 2 qui est solidaire de la came convexe 13 sur son côté externe 21, tandis que le plateau tibial 3 présente une piste 18 située à l'extérieur de l'empreinte 16. Dans cet exemple de réalisation l'espace 10 est compris entre les deux condyles 8 et 9 de l'élément fémoral 2 pour le passage du ligament croisé postérieur.

De la même manière que pour le condyle externe 8, le condyle interne 9 peut être solidaire sur son côté externe 22 d'une came convexe 13, tandis que le plateau tibial 3 présente une piste 18 située à l'extérieur de l'empreinte 17.

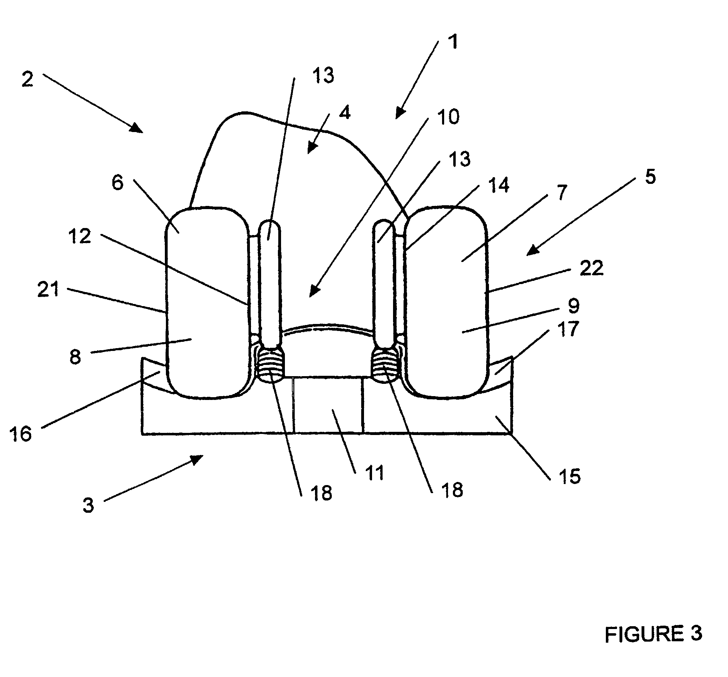

En figure 3 on a représenté une autre variante de la prothèse totale de genou 1 dont l'élément fémoral 2 comporte sur les côtés internes 12 et 14 de chaque condyle 8 et 9 une came convexe 13. Ainsi les deux cames convexes opposées 13 délimitent les dimensions de l'espace libre 10 pour le passage du ligament croisé postérieur. Chaque came 13 de l'élément fémoral 2 coopère avec une piste 18 ménagée entre et à proximité des empreintes 16 et 17 du plateau tibial 3 propre à recevoir les condyles 8 et 9.

La came convexe 13 représenté en figures 1 à 3, solidaire, soit du condyle externe 8, soit du condyle interne 9, soit des deux condyles 8 et 9, présente un profil en portion de tore permettant un petit jeu en rotation et évitant le phénomène de coupe.

On entend par un profil en portion de tore une surface de révolution engendrée par un cercle qui tourne autour d'un axe situé dans son plan et ne passant pas par son centre.

On note que la came convexe 13 peut être de manière plus générale une surface torique.

On entend par surface torique la surface engendrée par un cercle se déplaçant sur une ligne quelconque, le plan du cercle restant perpendiculaire à la ligne de déplacement.

Lorsque l'élément fémoral 2 comporte sur les côtés internes 12 et 14 de chaque condyle 8 et 9, une came 13, on constate que ce système ne change pas les appuis habituels du genou (figure 3).

De plus, l'élément fémoral 2 suivant l'invention, comportant une ou deux cames 13, permet un jeu de rotation par rapport à l'axe tibial.

En outre, l'élément fémoral 2 suivant l'invention, comportant une ou deux cames 13, permet de supprimer le conflit rotulien des prothèses de genou de l'art antérieur.

En effet, on remarque que la came convexe 13, vue de face, présente une largeur très faible, arrondie qui est très inférieure à celle prévue pour les condyles externe 8 et interne 9.

En fonction de la conservation ou non du ligament croisé postérieur la piste 18 recevant la came convexe 13 peut présenter des profils différents.

En effet, en figure 4 on a montré que la piste 18 comporte du côté de l'extrémité postérieure du plateau 3, une empreinte horizontale 19 se prolongeant vers l'extrémité antérieure dudit plateau par une butée courbe 20 qui se dresse à l'opposé du tibia en direction de l'élément fémoral 2. L'empreinte horizontale 19 est située en dessous des empreintes 16 et 17 recevant les condyles 8 et 9 de l'élément fémoral 2 (figure 4).

Le plateau tibial 3 peut être utilisé avec un élément fémoral 2 décrit en figure 1 à 3 pour constitué une prothèse totale du genou 1 avec conservation ou non du ligament croisé postérieur.

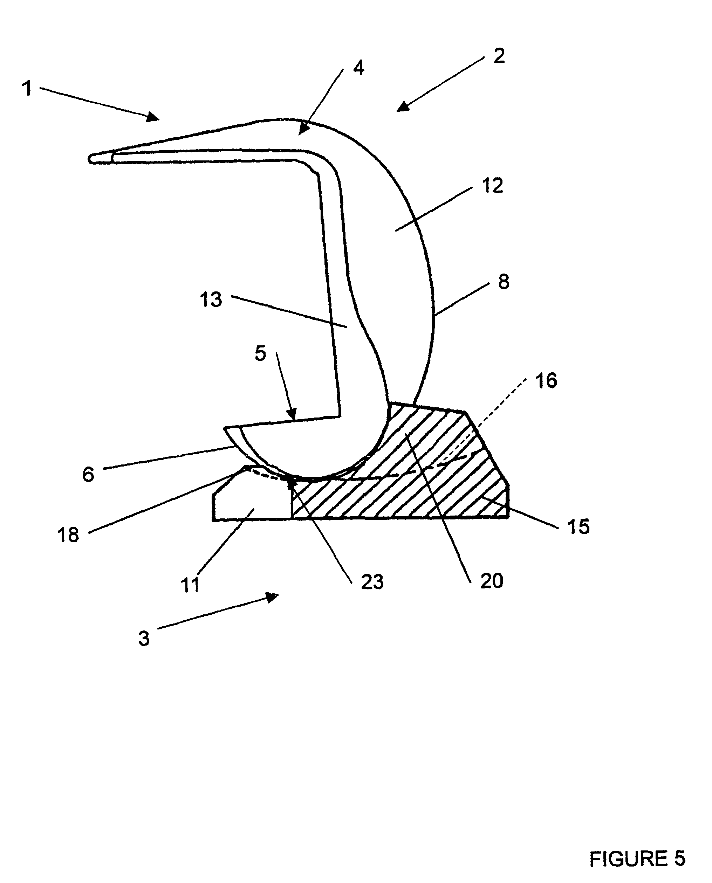

En figure 5 la piste 18 comporte du côté de l'extrémité postérieure du plateau tibial 3, c'est à dire du côté de l'échancrure 11, une première butée courbe 23 s'élevant légèrement au-dessus des empreintes 16 et 17 prévus pour les condyles 8 et 9.

La première butée 23 se prolonge vers l'extrémité antérieure dudit plateau par une seconde butée courbe 20 qui se dresse à l'opposé du tibia. C'est pourquoi la figure 5 ne montre pas une variante de la prothèse totale de genou suivant la présente invention.

Le plateau tibial 3 proposé en figure 5 peut être utilisé avec un élément fémoral 2 décrit en figure 1 à 3 pour permettre la conservation de ligaments postérieurs croisés, avec un passage automatique en fonctionnement postéro-stabilisé si le ligament vient à se détruire ultérieurement.

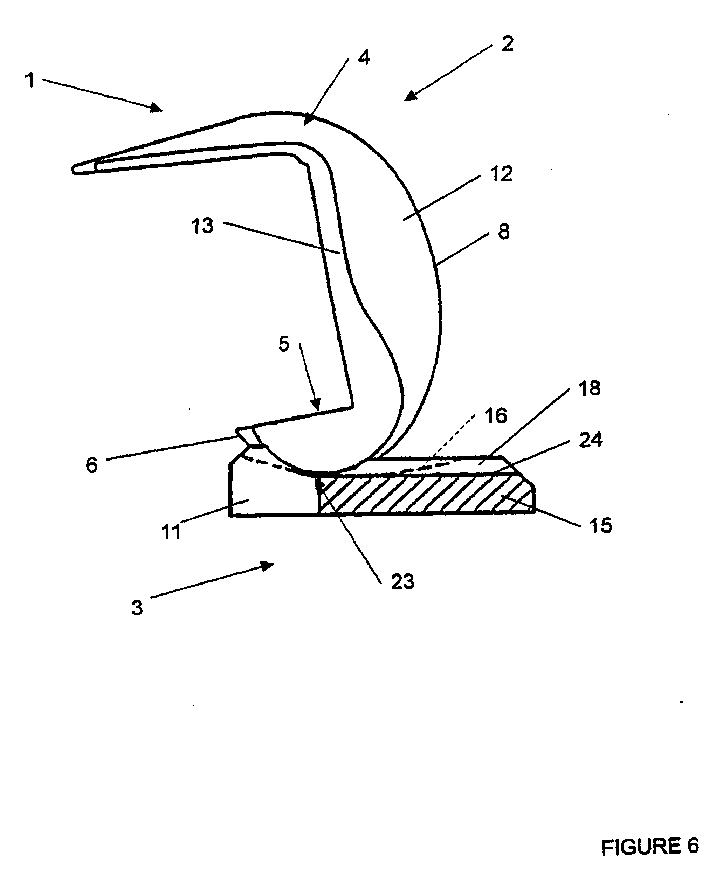

En figure 6 la piste 18 comporte du côté de l'extrémité postérieure du plateau tibial 3, c'est à dire au niveau de l'échancrure 11, la butée courbe 23 s'élevant légèrement au-dessus des empreintes 16 et 17, et se prolongeant vers l'extrémité antérieure dudit plateau par une empreinte horizontale 24 située en dessous desdites empreintes 16 et 17 pour les condyles 8 et 9 de l'élément fémoral 2.

Ainsi ce plateau tibial 3 peut être utilisé avec un élément fémoral 2 décrit précédemment en figures 1 à 3 pour constituer une prothèse totale du genou 1 avec la conservation de ligament croisé postérieur où le recul est maîtrisé par la butée 23 lors des déplacements de l'élément fémoral.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tout autre équivalent.

## Revendications

1. Prothèse totale de genou comprenant :
• Un élément fémoral (2) comportant deux condyles (8, 9), un externe, un interne et au moins une came convexe (13) solidaire des condyles (8, 9) pour assurer la stabilisation postérieure de la prothèse,
• Un plateau tibial (3) muni d'empreintes (16, 17) propre à recevoir les condyles (8, 9) et d'au moins une piste (18) disposée à proximité immédiate desdites empreintes afin de coopérer avec la came convexe (13),
• Un espace libre (10) et une échancrure (11) ménagés respectivement entre les condyles (8, 9) de l'élément fémoral (2) et les empreintes (16, 17) du plateau tibial (3) pour le passage du ligament croisé postérieur du genou,
**caractérisée en ce que** l'un au moins des côtés internes (12, 14) des condyles (8, 9) est solidaire d'une came convexe (13) réalisée sous la forme d'un troisième condyle et coopérant avec une piste (18) qui est délimitée par une butée courbe (20, 23) et au moins une empreinte horizontale (19, 24) entre les extrémités postérieures et antérieures du plateau tibial (3).

2. Prothèse totale du genou suivant la revendication 1, **caractérisée en ce que** le côté interne (12, 14) d'un condyle (8, 9) de l'élément fémoral (2) est solidaire d'une came convexe (13) réalisée sous la forme d'un troisième condyle de manière à délimiter les dimensions de l'espace libre (10) communiquant avec l'échancrure (11) du plateau tibial (3) pour le passage du ligament croisé postérieur du genou, tandis que la came convexe (13) coopère avec une piste (18) située entre et en dessous du niveau des empreintes (16, 17) recevant lesdits condyles (8, 9).

3. Prothèse totale du genou suivant la revendication 1, **caractérisée en ce que** les côtés internes (12, 14) de chaque condyle (8, 9) de l'élément fémoral (2) sont solidaires d'une came convexe (13) réalisée sous la forme d'un troisième condyle de manière à délimiter entre lesdites cames disposées à l'opposé l'une de l'autre les dimensions de l'espace libre (10) communiquant avec l'échancrure (11) du plateau tibial (3) pour le passage du ligament croisé postérieur du genou, tandis que chaque came convexe (13) coopère avec une piste (18) située entre et en dessous du niveau des empreintes (16,17) recevant lesdits condyles (8, 9).

4. Prothèse totale du genou suivant la revendication 1, **caractérisée en ce que** le plateau tibial (3) est pourvu d'au moins une piste (18) recevant la came convexe (13) correspondante de l'élément fémoral (2) et qui comporte du côté de l'extrémité postérieure dudit plateau une empreinte horizontale (19) située en dessous du niveau des empreintes (16, 17) propres à recevoir les condyles (8, 9), ladite empreinte (19) se prolongeant vers l'extrémité antérieure dudit plateau par une butée courbe (20) qui se dresse à l'opposé du tibia.

5. Prothèse totale du genou suivant la revendication 1, **caractérisée en ce que** le plateau tibial (3) est pourvu d'au moins une piste (18) recevant la came convexe (13) correspondante de l'élément fémoral (2) et qui comporte du côté de l'extrémité postérieure dudit plateau une première butée courbe (23) s'élevant légèrement au-dessus des empreintes (16, 17) propre à recevoir les condyles (8, 9), ladite première butée se prolongeant vers l'extrémité antérieure dudit plateau par une seconde butée courbe (20) qui se dresse à l'opposé du tibia.

6. Prothèse totale du genou suivant la revendication 1, **caractérisée en ce que** le plateau tibial (3) est pourvu d'au moins une piste (18) recevant la came convexe (13) correspondante de l'élément fémoral (2) et qui comporte du côté de l'extrémité postérieure dudit plateau une butée courbe (23) s'élevant légèrement au-dessus des empreintes (14, 17) propres à recevoir les condyles (8, 9), ladite butée se prolongeant vers l'extrémité antérieure dudit plateau par une empreinte horizontale (24) située en dessous du niveau desdites empreintes (16, 17).

7. Prothèse totale du genou suivant la revendication 1, **caractérisée en ce que** la came (13) présente un profil courbe s'étendant à partir du voile (6) jusqu'à l'aile antérieure (4) de l'élément fémoral (2).

8. Prothèse totale de genou suivant la revendication 1, **caractérisée en ce que** la came (13) présente un profil en portion de tore.

9. Prothèse totale de genou suivant la revendication 1, **caractérisée en ce que** la came (13) est une surface torique.

10. Prothèse totale de genou suivant la revendication 1, **caractérisée en ce que** la came (13) présente une largeur qui est inférieure à celle prévue pour les condyles externes (8) et internes (9).

## Claims

1. Total prosthesis of the knee comprising:
• A femoral element (2) having two condyles (8, 9), one external, the other internal, and at least one convex cam (13) made integral with the condyles (8, 9) in order to ensure posterior stabilization of the prosthesis,
• A tibial plateau (3) provided with recesses (16, 17) which receive the condyles (8, 9) and with at least one track (18) arranged in the immediate proximity of the said recesses in order to cooperate with the convex cam (13),
• A free space (10) and a notch (11) formed, respectively, between the condyles (8, 9) of the femoral element (2) and the recesses (16, 17) of the tibial plateau (3) for the passage of the posterior cruciate ligament of the knee,
**characterized in that** at least one of the internal sides (12, 14) of the condyles (8, 9) is integral with a convex cam (13) fashioned as a third condyle and cooperating with a track (18) which is delimited by a curved abutment (20, 23) and at least one horizontal recess (19, 24) between the posterior and anterior ends of the tibial plateau (3).

2. Total prosthesis of the knee according to Claim 1, **characterized in that** the internal side (12, 14) of one condyle (8, 9) of the femoral element (2) is integral with a convex cam (13) fashioned as a third condyle in such a way as to limit the dimensions of the free space (10) communicating with the notch (11) of the tibial plateau (3) for the passage of the posterior cruciate ligament of the knee, while the convex cam (13) works with a track (18) situated between and below the level of the recesses (16, 17) receiving the said condyles (8, 9).

3. Total prosthesis of the knee according to Claim 1, **characterized in that** the internal sides (12, 14) of each condyle (8, 9) of the femoral element (2) are integral with a convex cam (13) fashioned as a third condyle in such a way as to limit, between the said cams installed on opposite sides of it, the dimensions of the free space (10) communicating with the notch (11) of the tibial plateau (3) for the passage of the posterior cruciate ligament of the knee, while each convex cam (13) works with a track (18) situated between and below the level of the recesses (16, 17) receiving the said condyles (8, 9).

4. Total prosthesis of the knee according to Claim 1, **characterized in that** the tibial plateau (3) is fitted with at least one track (18) receiving the corresponding convex cam (13) of the femoral element (2) and which includes at the posterior extremity of the said plateau a horizontal recess (19) situated below the level of the recesses (16, 17) which receive the condyles (8, 9), the said recess (19) extending towards the anterior extremity of the said plateau via a curved abutment (20) which is raised against the tibia.

5. Total prosthesis of the knee according to Claim 1, **characterized in that** the tibial plateau (3) is fitted with at least one track (18) receiving the corresponding convex cam (13) of the femoral element (2) and which includes at the posterior extremity of the said plateau a first curved abutment (23) rising slightly above the recesses (16, 17) which receive the condyles (8, 9), the said first abutment extending towards the anterior extremity of the said plateau via a second curved abutment (20) which is raised against the tibia.

6. Total prosthesis of the knee according to Claim 1, **characterized in that** the tibial plateau (3) is fitted with at least one track (18) receiving the corresponding convex cam (13) of the femoral element (2) and which includes at the posterior extremity of the said plateau a curved abutment (23) rising slightly above the recesses (16, 17) which receive the condyles (8, 9), the said abutment extending towards the anterior extremity of the said plateau via a horizontal recess (24) situated below the level of the said recesses (16, 17).

7. Total prosthesis of the knee according to Claim 1, **characterized in that** the cam (13) has a curved shape extending from the camber (6) to the anterior wing (4) of the femoral element (2).

8. Total prosthesis of the knee according to Claim 1, **characterized in that** the cam (13) has a profile in the shape of a portion of a torus.

9. Total prosthesis of the knee according to Claim 1, **characterized in that** the cam (13) has a torus surface.

10. Total prosthesis of the knee according to Claim 1, **characterized in that** the cam (13) is less wide than the planned width of the external (8) and internal (9) condyles.

## Patentansprüche

1. Totalknieprothese, umfassend:
• ein Femoralelement (2), umfassend zwei Kondylen (8, 9), eine äußere und eine innere, und mindestens eine konvexe Nockenscheibe (13), die fest mit den Kondylen (8, 9) verbunden ist, um die hintere Stabilisierung der Prothese sicher zu stellen,
• eine Tibialplatte (3), die mit Vertiefungen (16, 17), die die Kondylen (8, 9) aufnehmen können, und mit mindestens einer Spur (18) versehen ist, die in unmittelbarer Nähe der Vertiefungen angeordnet ist, um mit der konvexen Nockenscheibe (13) zusammenzuwirken,
• einen Freiraum (10) und einen bogenförmigen Ausschnitt (11), die zwischen den Kondylen (8, 9) des Femoralelements (2) bzw. den Vertiefungen (16, 17) der Tibialplatte (3) für den Durchgang des hinteren Kreuzbandes des Knies vorgesehen sind,
**dadurch gekennzeichnet, dass** mindestens eine der Innenseiten (12, 14) der Kondylen (8, 9) fest mit einer konvexen Nockenscheibe (13) verbunden ist, die in Form einer dritten Kondyle ausgeführt ist und mit einer Spur (18), die durch einen gekrümmten Anschlag (20, 23) begrenzt ist, und mindestens einer horizontalen Vertiefung (19, 24) zwischen den hinteren und vorderen Enden der Tibialplatte (3) zusammenwirkt.

2. Totalknieprothese nach Anspruch 1, **dadurch gekennzeichnet, dass** die Innenseite (12, 14) einer Kondyle (8, 9) des Femoralelements (2) fest mit einer konvexen Nockenscheibe (13) verbunden ist, die in Form einer dritten Kondyle ausgeführt ist, um die Abmessungen des Freiraums (10), der mit dem bogenförmigen Ausschnitt (11) der Tibialplatte (3) für den Durchgang des hinteren Kreuzbandes des Knies in Verbindung steht, zu begrenzen, während die konvexe Nockenscheibe (13) mit einer Spur (18) zusammenwirkt, die zwischen den und unterhalb der Höhe der Vertiefungen (16, 17), die die Kondylen (8, 9) aufnehmen, angeordnet ist.

3. Totalknieprothese nach Anspruch 1, **dadurch gekennzeichnet, dass** die Innenseiten (12, 14) jeder Kondyle (8, 9) des Femoralelements (2) mit einer konvexen Nockenscheibe (13), die in Form einer dritten Kondyle ausgeführt ist, verbunden sind, um zwischen den einander gegenüber liegenden Nockenscheiben die Abmessungen des Freiraums (10) zu begrenzen, der mit dem bogenförmigen Ausschnitt (11) der Tibialplatte (3) für den Durchgang des hinteren Kreuzbandes des Knies in Verbindung steht, während jede konvexe Nockenscheibe (13) mit einer Spur (18) zusammenwirkt, die zwischen den und unterhalb der Höhe der Vertiefungen (16, 17), die die Kondylen (8, 9) aufnehmen, angeordnet ist.

4. Totalknieprothese nach Anspruch 1, **dadurch gekennzeichnet, dass** die Tibialplatte (3) mit mindestens einer Spur (18) versehen ist, die die entsprechende konvexe Nockenscheibe (13) des Femoralelements (2) aufnimmt und auf der Seite des hinteren Endes der Platte eine horizontale Vertiefung (19) umfasst, die unterhalb der Höhe der Vertiefungen (16, 17) angeordnet ist, die die Kondylen (8, 9) aufnehmen können, wobei sich die Vertiefung (19) zum vorderen Ende der Platte durch einen gekrümmten Anschlag (20) verlängert, der gegenüber der Tibia liegt.

5. Totalknieprothese nach Anspruch 1, **dadurch gekennzeichnet, dass** die Tibialplatte (3) mit mindestens einer Spur (18) versehen ist, die die entsprechende konvexe Nockenscheibe (13) des Femoralelements (2) aufnimmt und auf der Seite des hinteren Endes der Platte einen ersten gekrümmten Anschlag (23) aufweist, der leicht über die Vertiefungen (16, 17) ragt, die die Kondylen (8, 9) aufnehmen können, wobei sich der erste Anschlag zum vorderen Ende der Platte durch einen zweiten gekrümmten Anschlag (20) verlängert, der gegenüber der Tibia liegt.

6. Totalknieprothese nach Anspruch 1, **dadurch gekennzeichnet, dass** die Tibialplatte (3) mit mindestens einer Spur (18) versehen ist, die die entsprechende konvexe Nockenscheibe (13) des Femoralelements (2) aufnimmt und auf der Seite des hinteren Endes der Platte einen gekrümmten Anschlag (23) umfasst, der leicht über die Vertiefungen (16, 17) ragt, die die Kondylen (8, 9) aufnehmen können, wobei sich der Anschlag zum vorderen Ende der Platte durch eine horizontale Vertiefung (24) verlängert, die sich unterhalb der Höhe der Vertiefungen (16, 17) befindet.

7. Totalknieprothese nach Anspruch 1, **dadurch gekennzeichnet, dass** die Nockenscheibe (13) ein gekrümmtes Profil aufweist, das sich vom Velum (6) bis zum vorderen Flügel (4) des Femoralelements (2) erstreckt.

8. Totalknieprothese nach Anspruch 1, **dadurch gekennzeichnet, dass** die Nockenscheibe (13) ein Profil in Form eines Torusabschnittes aufweist.

9. Totalknieprothese nach Anspruch 1, **dadurch gekennzeichnet, dass** die Nockenscheibe (13) eine torische Oberfläche aufweist.

10. Totalknieprothese nach Anspruch 1, **dadurch gekennzeichnet, dass** die Nockenscheibe (13) eine Breite aufweist, die geringer ist als jene, die für die äußere (8) und innere Kondyle (9) vorgesehen ist.
